# EUROPÄISCHE PATENTANMELDUNG

(11) **EP 3 960 265 A1**
(43) Veröffentlichungstag der Anmeldung: **02.03.2022**
(21) Anmeldenummer: 20020393.3
(22) Anmeldetag: 31.08.2020
(51) Int. Cl.: B01D 3/14, B01D 5/00, B01D 53/14, C07C 29/151, C07C 41/09, C07C 41/34, C07C 41/40, C07C 41/42, C07C 43/04, C07C 31/04

(54) **VERFAHREN UND ANLAGE ZUR HERSTELLUNG VON DIMETHYLETHER**

(71) Anmelder: Linde GmbH, 82049 Pullach (DE)
(72) Erfinder: Peschel, Andreas, 82515 Wolfratshausen (DE)
(74) Vertreter: Fischer, Werner

(57) **Zusammenfassung**

Es wird ein Verfahren zur Herstellung von Dimethylether (Abkürzung: DME) aus Synthesegas vorgeschlagen, wobei ein Einsatz (c), der unter Verwendung von geshiftetem und/oder ungeshiftetem Synthesegas (b) gebildet wird, unter Bildung eines Produktstroms (d) einer katalytischen Umsetzung unterworfen wird. Der Produktstrom (d) wird einer ersten Auftrennung unterworfen, wobei ein Gasgemisch (k) durch zumindest teilweise Abtrennung von Methanol und/oder Wasser aus dem Produktstrom (d) gebildet wird und das Gasgemisch (k) auf einem ersten Druckniveau durch Abkühlung von einem ersten auf ein zweites Temperaturniveau teilweise kondensiert wird. Ein auf dem zweiten Temperaturniveau gasförmig bleibender Anteil (s) des Gasgemischs (k) wird in einer Absorptionskolonne (16) mit einem überwiegend Dimethylether enthaltenden Rücklauf (v) gewaschen, wobei der überwiegend Dimethylether enthaltende Rücklauf (v) zumindest teilweise aus einem bei der Abkühlung kondensierten Anteil des Gasgemischs (k) gebildet wird. Der in der Absorptionskolonne (16) nicht ausgewaschene gasförmige Anteil (x) des Gasgemischs (k) wird zumindest teilweise als Recyclingstrom (j) in den Einsatz (c) zurückgeführt und ein Dimethyletherprodukt (z) wird unter Verwendung des bei der Abkühlung kondensierten Anteils (l, q, r) des Gasgemischs (k) gebildet.

Eine Trennanlage zur trenntechnischen Bearbeitung des Gasgemischs sowie eine Anlage zur Durchführung des gesamten erfindungsgemäßen Verfahrens werden ebenfalls angegeben.

## Beschreibung

Die Erfindung betrifft ein Verfahren zur Herstellung von Dimethylether (DME) sowie eine Anlage zur Durchführung eines solchen Verfahrens und eine Anlage zur trenntechnischen Bearbeitung eines Zwischenprodukts des Verfahrens.

### Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas ist ein Gasgemisch, das zumindest Kohlenstoffmonoxid und Wasserstoff in wechselnden Anteilen, die zusammengenommen jedoch den überwiegenden Teil des Gasgemischs ausmachen, enthält. Synthesegas kann auch mittels anderer Verfahren, beispielsweise durch Pyrolyse von Kohle, Öl, kohlenstoffhaltigen Abfällen, Biomasse oder anderen kohlenstoffhaltigen Ausgangsstoffen, durch Trockenreformierung (DryReforming) von Erdgas mit Kohlenstoffdioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden. Das Synthesegas wird anschließend entweder in einer zweistufigen Reaktion zu Methanol und anschließend zu Dimethylether oder in einer einstufigen Reaktion direkt zu Dimethylether konvertiert.

Die Synthese von Dimethylether aus Synthesegas ist thermodynamisch und wirtschaftlich vorteilhaft gegenüber einer Synthese aus Methanol.

Die vorliegende Erfindung betrifft insbesondere die einstufige Synthese von Dimethylether, wobei unter einer "einstufigen" Synthese eine Synthese verstanden wird, bei der sämtliche Reaktionen in einunddemselben Reaktor ablaufen. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 100 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält neben Dimethylether nicht umgesetzte Komponenten des Synthesegases sowie weitere Reaktionsprodukte. Typischerweise weist der Produktstrom neben Dimethylether zumindest Methanol, Wasser, Kohlenstoffdioxid, Kohlenstoffmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlenstoffdioxid und leichter als Kohlenstoffdioxid siedende Komponenten wie Wasserstoff und Kohlenstoffmonoxid enthalten. Zur spezifikationsgerechten Gewinnung von Dimethylether müssen diese zumindest teilweise abgetrennt werden. Hierzu verwendete Verfahren erweisen sich jedoch insbesondere unter energetischen Aspekten als unbefriedigend.

Zur Gewinnung von Dimethylether aus dem Produktstrom muss dieser auf Temperaturen deutlich unter 0 °C abgekühlt werden. Hierbei kann es erforderlich sein, vor der Abkühlung größere Mengen an Methanol und Wasser abzutrennen. Aus der WO 2015/104290 A1 sind jedoch auch Verfahren bekannt, bei denen eine derartige Abtrennung nicht erforderlich ist.

Es soll ein verbessertes Recyclingkonzept für nicht umgesetztes Synthesegas angegeben werden.

### Offenbarung der Erfindung

Diese Aufgabe wird durch ein Verfahren zur Herstellung von Dimethylether (DME), eine Anlage zur trenntechnischen Bearbeitung eines Gasgemischs sowie eine Anlage zur Durchführung eines solchen Verfahrens mit den Merkmalen der jeweiligen unabhängigen Patentansprüche gelöst. Vorteilhafte Weiterbildungen der Erfindung sind Gegenstand der abhängigen Patentansprüche sowie der folgenden Beschreibung. Vor der Erläuterung der Merkmale und Vorteile der vorliegenden Erfindung werden deren Grundlagen und die verwendeten Begriffe erläutert.

Ein Fluid (die Bezeichnung "Fluid" wird nachfolgend auch kurz für entsprechende Ströme, Fraktionen usw. verwendet) ist von einem anderen Fluid (das auch als Ausgangsfluid bezeichnet wird) "abgeleitet" oder aus einem solchen Fluid "gebildet", wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch "gebildet" werden, dass er aus einem Speicherbehälter abgezogen wird.

Fluide können im hier verwendeten Sprachgebrauch reich oder arm an einer oder mehreren enthaltenen Komponenten sein, wobei "reich" für einen Gehalt von wenigstens >50%, 75%, 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Sie können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist "angereichert", wenn es zumindest den 1,1-fachen, 1,5-fachen, 2-fachen, 5-fachen, 10-fachen 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein "überwiegend" eine oder mehrere Komponenten enthaltendes Fluid enthält diese eine oder mehreren Komponenten zu wenigstens >50%, 75%, 90%, 95%, 98% oder 99% bzw. ist reich an diesen.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte verwendet werden müssen, um ein erfinderisches Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Unterschiedliche Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Destillationssäule mittels eines Sumpfverdampfers erwärmt, so dass kontinuierlich ein Teil der Sumpfflüssigkeit verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, hier als Kopfgas bezeichnet, eingespeist, zu einem Teil zu einem Kondensat verflüssigt und als flüssiger Rücklauf am Kopf der Destillationssäule aufgegeben wird. Ein Teil des aus dem Kopfgas erhaltenen Kondensats kann anderweitig verwendet werden.

Im Gegensatz zu einer Destillationssäule verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen. Am Kopf einer solchen Absorptionskolonne wird ein gasförmiges Fluid erhalten, das als "Kopfprodukt" aus dieser abgezogen werden kann. Im Sumpf der Absorptionskolonne scheidet sich eine Flüssigkeit ab, die als "Sumpfprodukt" abgezogen werden kann. Die Gasphase wird in der Absorptionskolonne bezüglich einer oder mehrerer Komponenten abgereichert, welche in das Sumpfprodukt übergehen.

Zur Auslegung und spezifischen Ausgestaltung von Destillationssäulen und Absorptionskolonnen sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

Ist nachfolgend kurz von einer "Synthese" von Dimethylether die Rede, wird hierunter ein Verfahren verstanden, bei dem ein Synthesegas bzw. Syngas enthaltender Einsatz, also ein Gasgemisch, das in geeigneten Anteilen zumindest Kohlenstoffmonoxid und Wasserstoff enthält, zu einem entsprechenden Dimethylether enthaltenden Produktstrom umgesetzt wird. Der Anteil an Wasserstoff in dem Synthesegas kann dabei beispielsweise unter Verwendung einer Wassergas-Shift-Reaktion erhöht werden. Dabei reagiert in dem Synthesegas vorhandenes Kohlenstoffmonoxid mit zu diesem Zweck zugegebenem Wasser(dampf) zu Kohlenstoffdioxid, das gegebenenfalls abgetrennt werden kann. Ein derart nachbehandeltes Synthesegas wird auch als geshiftetes Synthesegas bezeichnet. Ein entsprechender Produktstrom der Synthese von Dimethylether enthält aufgrund der nicht vollständigen Reaktion und aufgrund des Auftretens von Nebenreaktionen bei der Synthese von Dimethylether, insbesondere in Abhängigkeit von den Charakteristika der verwendeten Katalysatoren und den jeweiligen Gehalten der Komponenten des Synthesegases, nicht ausschließlich Dimethylether sondern weitere Verbindungen. Diese sind zumindest Methanol, Wasser, Kohlenstoffdioxid, Kohlenstoffmonoxid und Wasserstoff, jedoch typischerweise auch geringere Mengen Methan, Ethan, organische Säuren und höhere Alkohole. Diese genannten weiteren Verbindungen müssen, wie erwähnt, abgetrennt werden. Die Abtrennung erfolgt einerseits, um nachfolgende Trennschritte zu ermöglichen, und andererseits, um Dimethylether in der geforderten Reinheit, also "spezifikationsgerecht" zu gewinnen.

### Vorteile der Erfindung

In dem erfindungsgemäßen Verfahren wird, wie erläutert, ein Gasgemisch trenntechnisch bearbeitet, das aus einem Produktstrom eines Reaktors zur Synthese von Dimethylether aus Synthesegas gebildet wird, und das zumindest Dimethylether, Kohlenstoffdioxid und zumindest eine weitere, leichter als Kohlenstoffdioxid siedende Komponente enthält. Bei solchen, leichter als Kohlenstoffdioxid siedenden Komponenten kann es sich insbesondere um Komponenten wie Kohlenstoffmonoxid und Wasserstoff handeln. Wie erwähnt, sind andere, ebenfalls leichter als Kohlenstoffdioxid siedende Komponenten, beispielsweise Methan, in einem derartigen Gasgemisch in geringerer Menge ebenfalls enthalten.

Zur Erhöhung der Ausbeute wird erfindungsgemäß nicht umgesetztes Synthesegas zumindest teilweise als Recyclingstrom zu der Synthese zurückgeführt und dieser erneut unterworfen.

Der in der Synthese eingesetzte Katalysator ist empfindlich gegenüber Kohlenstoffdioxid-Konzentrationen, die 8 % oder auch 4 % der Stoffmenge des Einsatzgemischs übersteigen. Da die Menge an Kohlenstoffdioxid, die in der Synthese gebildet wird, von der Zusammensetzung des Einsatzgemischs abhängt, werden erfindungsgemäß geshiftetes und ungeshiftetes Synthesegas im benötigten Verhältnis gemischt. Beispielsweise wird ein Verhältnis von Wasserstoff zu Kohlenstoffmonoxid im Bereich von 1.15 bis 1.5 eingestellt. Geshiftetes Synthesegas ist dabei insbesondere reich an Wasserstoff, ungeshiftetes ist insbesondere reich an Kohlenstoffmonoxid. Je kleiner das Verhältnis der Anteile von Wasserstoff zu Kohlenstoffmonoxid im Einsatzgemisch, desto mehr Kohlenstoffdioxid entsteht bei der Synthese. Dies ist anhand folgender stark vereinfachter Reaktionsgleichungen unschwer nachzuvollziehen:

| | |
|---|---|
| 3 H₂ + 3 CO → DME + CO₂ | (H₂ : CO = 1) |
| 4 H₂ + 2 CO → DME + H₂O | (H₂ : CO = 2) |

Wird nun aus dem Recyclingstrom Kohlenstoffdioxid entfernt, bevor dieser zur Synthese zurückgeleitet wird, ist auch bei niedrigen Verhältnissen von Wasserstoff zu Kohlenstoffmonoxid ein Zurückführen der nicht verbrauchten Bestandteile des Einsatzes zur Synthese möglich, ohne den Katalysator mit für diesen schädlichem bzw. störendem Kohlenstoffdioxid zu belasten. Daher kann das Verfahren mit niedrigerem Anteil an geshiftetem Synthesegas betrieben werden, was wirtschaftlich vorteilhaft ist, da eine entsprechende Anlage zum Shiften damit kleiner ausfallen kann oder gar ganz wegfallen kann. Das Verfahren kann somit mit einem höheren Anteil oder ganz mit kostengünstigem ungeshiftetem Synthesegas betrieben werden.

Vorteilhafterweise wird nicht das gesamte unverbrauchte Synthesegas als der Recyclingstrom zu der Synthese zurückgeführt, da sich durch ein derartiges geschlossenes Kreislaufsystem Verunreinigungen, die beispielsweise über den Frischeinsatz in das Verfahren eingeschleust werden können, anreichern. Solche Verunreinigungen können beispielsweise Argon, Stickstoff, Sauerstoff, Helium oder andere inerte Gase sowie beispielsweise Alkohole, Aldehyde, Ketone, Ester, Ether, Olefine, Alkane und andere Kohlenwasserstoffe, die als Nebenprodukte aus der Synthese oder der Synthesegas-Bereitstellung resultieren können, sein.

Eine "leichter als Kohlenstoffdioxid siedende" Komponente besitzt einen niedrigeren Siedepunkt als Kohlenstoffdioxid. Es sei an dieser Stelle darauf hingewiesen, dass bei den erfindungsgemäß eingesetzten Druckniveaus (das nachfolgend erläuterte "erste" Druckniveau liegt oberhalb des Tripelpunkts von Kohlenstoffdioxid) Kohlenstoffdioxid auch in der flüssigen Phase vorliegen kann.

Erfindungsgemäß wird das Gasgemisch auf dem ersten Druckniveau ausgehend von einem ersten Temperaturniveau auf ein zweites Temperaturniveau abgekühlt. Dies kann vorteilhafterweise über ein oder mehrere Zwischentemperaturniveaus und unter Abscheidung eines oder mehrerer Kondensate aus dem Gasgemisch erfolgen, es ist jedoch auch eine einstufige Abkühlung möglich.

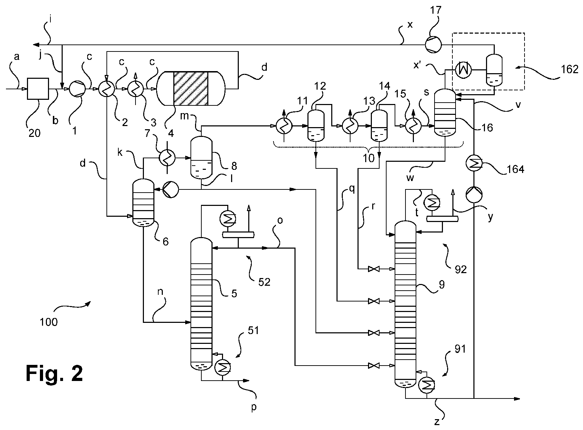

Wenn zuvor Kondensate abgeschieden werden, enthalten diese überwiegend Dimethylether und Kohlenstoffdioxid, da das verwendete Gasgemisch erfindungsgemäß durch eine vorherige Abtrennung von Methanol und Wasser aus dem Produktstrom arm an schwerer siedenden Komponenten wie Methanol und Wasser ist. Hierzu kann beispielsweise eine Anordnung zum Einsatz kommen, wie sie in der Figur 2 gezeigt ist. Insbesondere kann das der Abkühlung unterworfene Gasgemisch frei oder weitgehend frei von Methanol und Wasser sein. Bei Strömen, die Wasser und/oder Methanol enthalten, ist jedoch darauf zu achten, dass weder Methanol noch Wasser alleine in einem entsprechenden Gasgemisch enthalten sind. Wasser ohne einen zusätzlichen Anteil Methanol würde bei den eingesetzten tiefen Temperaturen ausfrieren, weil die Frostschutzwirkung des Methanols fehlen würde. Umgekehrt würde jedoch auch "trockenes" Methanol zu Nachteilen führen, weil dieses die verwendeten Wärmetauscher schädigen könnte. Als Frostschutzmittel kommt für die Wasser enthaltenden Ströme neben Methanol auch Dimethylether jeweils alternativ oder ergänzend in Frage.

Ein auf dem zweiten Temperaturniveau gasförmig bleibender Anteil des Gasgemischs wird in einer Absorptionskolonne unter Gewinnung eines Kopfprodukts und eines Sumpfprodukts mit einem überwiegend Dimethylether enthaltenden Rücklauf gewaschen. Dies dient dazu, in dem gasförmig bleibenden Anteil des Gasgemischs noch enthaltenes Kohlenstoffdioxid in das Sumpfprodukt auszuwaschen, so dass das Kopfprodukt nach Möglichkeit weitgehend frei von Kohlenstoffdioxid ist. Das Kopfprodukt besteht überwiegend aus der zumindest einen auf dem ersten Druckniveau leichter als Kohlenstoffdioxid siedenden Komponente. Das Kopfprodukt enthält vorzugsweise kein Kohlenstoffdioxid oder allenfalls geringe Mengen, ist also zumindest arm an Kohlenstoffdioxid im oben erläuterten Sinn. Erfindungsgemäß wird das Kopfprodukt aus der Absorptionskolonne zumindest teilweise zu der Synthese zurückgeführt. Es ist daher, wie eingangs erläutert, wichtig, dass möglichst wenig Kohlenstoffdioxid in diesem Recyclingstrom enthalten ist, da dieses den in der Synthese eingesetzten Katalysator schädigen kann. Die erfindungsgemäße Abtrennung von Kohlenstoffdioxid aus diesem Recyclingstrom ermöglicht dementsprechend längere Katalysator-Lebensdauern bzw. -Einsatzzyklen.

In einigen Ausführungsformen kann auch vorgesehen sein, gezielt einen bestimmten Anteil an Kohlenstoffdioxid in dem Recyclingstrom einzustellen, beispielsweise dergestalt, dass ein Anteil von ca. 2 % bis ca. 6 % Kohlenstoffdioxid am Eingang der Synthese vorliegt, was sich positiv auf die Reaktionsgeschwindigkeit in der Synthese auswirken kann.

Es kann auch vorteilhaft sein, einen Frischeinsatz über die Absorptionskolonne in die Synthese einzuspeisen, um den Kohlenstoffdioxidanteil in dem Einsatzstrom gezielt einzustellen. In einem solchen Fall wird der Frischeinsatz vor der Absorptionskolonne getrocknet.

Der überwiegend Dimethylether enthaltende Rücklauf wird erfindungsgemäß aus einem bei der Abkühlung auf das zweite Temperaturniveau flüssig abgeschiedenen Anteil des Gasgemischs gebildet. Hierzu wird eine Dimethylether-Kohlenstoffdioxid-Destillationssäule verwendet. Werden bei der Abkühlung ein oder mehrere Kondensat(e) abgeschieden, werden diese oder aus diesen gebildete Ströme zumindest zum Teil in die Dimethylether-Kohlenstoffdioxid-Destillationssäule eingespeist. In die Dimethylether-Kohlenstoffdioxid-Destillationssäule kann auch das Sumpfprodukt aus der Absorptionskolonne oder ein hieraus gebildeter Strom zumindest zum Teil eingespeist werden.

Unter einer "Dimethylether-Kohlenstoffdioxid-Destillationssäule" wird hier eine Destillationssäule verstanden, die so ausgebildet ist und betrieben wird, dass sich in ihr aus Fluiden, die Dimethylether und Kohlenstoffdioxid enthalten, Fluide gewinnen lassen, die einerseits an Dimethylether und andererseits an Kohlenstoffdioxid angereichert und an der jeweils anderen Komponente abgereichert sind. Die Fachperson wählt die spezifische Ausgestaltung einer Dimethylether-Kohlenstoffdioxid-Destillationssäule (wie beispielsweise Art und Anzahl der Einbauten) und die Betriebsbedingungen (wie beispielsweise Betriebsdruck, Heiz- und Kühlfluide im Sumpfverdampfer und Kopfkondensator usw.) insbesondere auf Grundlage der Siedepunktsunterschiede von Dimethylether und Kohlenstoffdioxid aus.

Ein wesentlicher Aspekt der vorliegenden Erfindung ist die geeignete Einstellung der Betriebsbedingungen der Dimethylether-Kohlenstoffdioxid-Destillationssäule. Diese sind vorteilhafterweise derart gewählt, dass ein überwiegend Kohlenstoffdioxid enthaltendes Kopfgas, das vom Kopf der Dimethylether-Kohlenstoffdioxid-Destillationssäule abgezogen wird, oberhalb der Schmelztemperatur von Kohlenstoffdioxid verflüssigt werden kann. Ein entsprechend erhaltenes Kondensat sollte in ausreichender Menge zur Verfügung stehen, um als Rücklauf für die Dimethylether-Kohlenstoffdioxid-Destillationssäule verwendet zu werden. Ein Teil der Sumpfflüssigkeit der Dimethylether-Kohlenstoffdioxid-Destillationssäule wird als Rücklauf für die Absorptionskolonne zur Verfügung gestellt, wie bereits teilweise erläutert.

Vorteilhafterweise wird die Dimethylether-Kohlenstoffdioxid-Destillationssäule derart betrieben, dass sich an ihrem Kopf ein überwiegend Kohlenstoffdioxid enthaltendes Kopfgas und in ihrem Sumpf eine an Dimethylether angereicherte Sumpfflüssigkeit bilden. Die Sumpfflüssigkeit enthält überwiegend Dimethylether, wenn die in die Dimethylether-Kohlenstoffdioxid-Destillationssäule eingespeisten Fluide (beispielsweise die erläuterten Kondensat(e) und das Sumpfprodukt aus der Absorptionssäule) überwiegend Kohlenstoffdioxid und Dimethylether enthalten. Enthalten letztere auch Wasser und Methanol, insbesondere in geringem Anteil, gehen diese ebenfalls in die Sumpfflüssigkeit der Dimethylether-Kohlenstoffdioxid-Destillationssäule über.

Die Erfindung ermöglicht damit durch die Verwendung des dimethyletherreichen flüssigen Rücklaufs auf die Absorptionskolonne eine Verringerung des Anteils an Kohlenstoffdioxid in dem zur Synthese zurückgeführten nicht umgesetzten Synthesegas. Als der überwiegend Dimethylether enthaltende Rücklauf wird dabei ein Teil eines Produktstroms verwendet, der aus zumindest einem Teil des Sumpfprodukts der Dimethylether-Kohlenstoffdioxid-Destillationssäule gebildet wird. Vorzugsweise enthält dieses Sumpfprodukt den gesamten Dimethylether, der in der Synthese hergestellt wurde oder dessen überwiegenden Anteil. Kopfseitig wird der Dimethylether-Kohlenstoffdioxid-Destillationssäule das bereits erwähnte Kopfgas entnommen, das überwiegend aus Kohlenstoffdioxid bestehen kann. Der Teil des Kopfgases, der nicht als Rücklauf für die Dimethylether-Kohlenstoffdioxid-Destillationssäule verwendet wird, kann zur Synthesegasbereitstellung rezykliert werden und/oder in gasförmiger oder kondensierter Form aus der Anlage ausgeschleust werden, um entweder als zusätzliches Wertprodukt vermarktet oder als Abfall verworfen zu werden. In den beiden letzteren Fällen kann eine Nachbehandlung des Kohlenstoffdioxids vorgesehen sein, beispielsweise zur Aufreinigung um Produktspezifikationen einzustellen oder im Sinne einer, beispielsweise gesetzlich vorgeschriebenen, Aufbereitung, beispielsweise für Carbon-Capture-and-Storage (CCS) Anwendungen. Insbesondere in Fällen, in denen das Synthesegas unter Verwendung von Kohlenstoffdioxid bereitgestellt wird, beispielsweise durch ein Elektrolyseverfahren, kann vorteilhafterweise die erwähnte Rückführung von Kohlenstoffdioxid zu der Bereitstellung von Synthesegas vorgesehen sein.

Wie eingangs erläutert, wird vorteilhafterweise nicht das gesamte, von Kohlenstoffdioxid befreite Kopfgas aus der Absorptionskolonne zu der Synthese zurückgeführt. Somit wird vorteilhafterweise ein Teil davon als Purgestrom aus dem Verfahren ausgeschleust. Da das Kopfgas aus der Absorptionskolonne noch relevante Mengen an Dimethylether enthalten kann, ist besonders bevorzugt vorgesehen, den Purgestrom einer zusätzlichen Wäsche mit flüssigem Kohlenstoffdioxid zu unterziehen, um möglichst große Anteile des darin enthaltenen Dimethylethers in die Dimethylether-Kohlenstoffdioxid-Destillationssäule zurückzuführen. Für diese Wäsche kann vorteilhafterweise ein Teil des kondensierten Kopfgases aus der Dimethylether-Kohlenstoffdioxid-Destillationssäule verwendet werden. Dies hat den zusätzlichen Vorteil, dass keine verfahrensfremden Ströme verwendet werden, was zusätzliche Verunreinigungsquellen minimiert.

Die Abkühlung auf das zweite Temperaturniveau erfolgt vorteilhafterweise mittels vorhandener Kältemittel, beispielsweise C2-Kältemittel (z.B. flüssigem Ethan, Ethylen oder einem Äquivalent zur Erreichung einer entsprechenden Temperatur), DME oder auch Kohlenstoffdioxid. Eine vorige stufenweise Abkühlung auf Zwischentemperaturniveaus, falls durchgeführt, erfolgt beispielsweise mit Wasser, Wasser/Glykol-Mischungen, einem C3-Kältemittel (z.B. flüssigem Propan, Propylen oder einem Äquivalent), DME oder auch Kohlenstoffdioxid. Dabei beträgt das erste Temperaturniveau vorteilhafterweise 10 bis 50 °C, insbesondere 20 bis 40 °C. Das zweite Temperaturniveau liegt vorteilhafterweise 0,5 bis 20 °C, insbesondere 1 bis 10 °C oberhalb der Schmelztemperatur von Kohlenstoffdioxid bei dem ersten Druckniveau.

Das erfindungsgemäß vorgeschlagene Verfahren erweist sich als wirtschaftlich bedeutend günstiger als herkömmliche Verfahren, so dass durch die erfindungsgemäßen Maßnahmen eine vorteilhafte Trennung gegenüber Trennverfahren erzielt wird, wie sie aus dem Stand der Technik bekannt sind. Durch die erfindungsgemäße Rückführung des an Kohlenstoffdioxid abgereicherten Recyclingstroms kann der Anteil an geshiftetem Synthesegas im Frischeinsatz reduziert werden, da eine Erhöhung des Kohlenstoffdioxidanteils in dem Produktstrom nicht zu einer katalysatorschädigenden Erhöhung des Kohlenstoffdioxidanteils im Einsatz führt, wie eingangs bereits erläutert.

Die vorliegende Erfindung eignet sich insbesondere für Verfahren, bei denen der Produktstrom aus dem zur Synthese von Dimethylether aus Synthesegas verwendeten Reaktor auf einem Druckniveau von 20 bis 100 bar, insbesondere auf einem Druckniveau von 30 bis 80 bar (dem "ersten Druckniveau") bereitgestellt wird. Der Produktstrom wird, wie bereits erwähnt, unter Verwendung einer weiteren Absorptionskolonne, auf diesem ersten Druckniveau weitgehend von Methanol und Wasser befreit. Die Abtrennung von Methanol und/oder Wasser kann also unter Druck erfolgen, eine vorherige Entspannung, die dann eine erneute energieaufwendige Druckbeaufschlagung erfordern würde, ist nicht notwendig. Das aus dem Produktstrom erhaltene Gasgemisch wird also nach dem Verlassen des Reaktors zur Synthese von Dimethylether und vor der erfindungsgemäßen trenntechnischen Bearbeitung nicht entspannt. Eine energieaufwendige erneute Verdichtung kann daher entfallen.

Das erfindungsgemäße Verfahren kann mit Produktströmen unterschiedlichster Zusammensetzung, wie sie im Rahmen der Synthese anfallen, zum Einsatz kommen. Entsprechende Produktströme enthalten beispielsweise 2 bis 50 Molprozent, insbesondere 5 bis 30 Molprozent Dimethylether, 0,1 bis 20 Molprozent, insbesondere 0,7 bis 10 Molprozent Methanol, 0,1 bis 20 Molprozent, insbesondere 0,8 bis 10 Molprozent Wasser, 1 bis 50 Molprozent, insbesondere 3 bis 30 Molprozent Kohlenstoffdioxid, 0,1 bis 25 Molprozent, insbesondere 1 bis 11 Molprozent Kohlenstoffmonoxid und 5 bis 90 Molprozent, insbesondere 20 bis 80 Molprozent Wasserstoff. Nach der Abtrennung von Wasser und Methanol, ist das Gasgemisch vorzugsweise arm an bzw. frei von Wasser und Methanol.

Entsprechende Produktströme können ferner geringere Anteile an weiteren Komponenten, beispielsweise Methan, Ethan, organische Säuren und höhere Alkohole enthalten, wie erwähnt. Entsprechende Gemische werden insbesondere in Verfahren erhalten, bei denen eine einstufige Synthese von Dimethylether vorgenommen wird.

Wie bereits erwähnt, wird die Dimethylether-Kohlenstoffdioxid-Destillationssäule im Rahmen der vorliegenden Erfindung vorteilhafterweise derart betrieben, dass ein überwiegend Kohlenstoffdioxid enthaltendes Kopfgas, das vom Kopf der Dimethylether-Kohlenstoffdioxid-Destillationssäule abgezogen wird, oberhalb der Schmelztemperatur von Kohlendioxid verflüssigt werden kann. Hierbei wird die Dimethylether-Kohlenstoffdioxid-Destillationssäule vorteilhafterweise auf einem zweiten Druckniveau betrieben, das unterhalb des ersten Druckniveaus liegt. Das zweite Druckniveau liegt beispielsweise bei 10 bis 40 bar, insbesondere bei 15 bis 30 bar.

Die Sumpfflüssigkeit kann der Dimethylether-Kohlenstoffdioxid-Destillationssäule als dimethyletherreicher Strom entnommen werden, der einen Dimethylethergehalt von wenigstens 90 Molprozent, insbesondere wenigstens 95 Molprozent oder wenigstens 98,5 Molprozent aufweist. Die jeweiligen Gehalte richten sich nach den Betriebsbedingungen der Dimethylether-Kohlenstoffdioxid-Destillationssäule und deren Konfiguration. Sie können der jeweils geforderten Spezifikation (Reinheit) eines entsprechend erhaltenen Produktstroms angepasst werden. Aus der Sumpfflüssigkeit der Dimethylether-Kohlenstoffdioxid-Destillationssäule wird auch ein Dimethyletherprodukt, das das eigentliche Zielprodukt des Verfahrens darstellt, gewonnen.

Wie erwähnt, besteht der flüssige Rücklauf, der auf die Absorptionskolonne aufgegeben wird, überwiegend aus Dimethylether. Vorteilhaft sind beispielsweise Dimethylethergehalte von wenigstens 90 Molprozent, insbesondere wenigstens 95 Molprozent oder wenigstens 98,5 Molprozent.

Das Verfahren ermöglicht eine Verringerung des Anteils an Kohlenstoffdioxid in der letzten Kondensationsstufe durch die Verwendung der Absorptionskolonne. Der aus dem Kopfgas der Absorptionskolonne gebildete, überwiegend Kohlenstoffmonoxid und Wasserstoff enthaltende Recyclingstrom weist dabei vorteilhafterweise einen Kohlenstoffdioxidgehalt von höchstens 10, höchstens 7 Molprozent, insbesondere höchstens 5 Molprozent, höchstens 1 Molprozent oder höchstens 0,5 Molprozent auf. Entsprechende Gehalte können auch im Kopfstrom der Absorptionskolonne erhalten werden.

Zur Verringerung von Verlusten an Dimethylether ist vorteilhafterweise vorgesehen, das Kopfgas der Absorptionskolonne zumindest teilweise zu kondensieren und in den Kopfbereich der Absorptionskolonne zurückzuführen.

Eine Trennanlage, die zur trenntechnischen Bearbeitung des mehrfach erläuterten Gasgemischs eingerichtet ist, ist ebenfalls Gegenstand der vorliegenden Erfindung. Das Gasgemisch wird aus einem Produktstrom eines Reaktors zur Synthese von Dimethylether aus Synthesegas gebildet. Die Trennanlage weist sämtliche Mittel auf, die sie zur Durchführung des zuvor erläuterten Verfahrens befähigen. Eine derartige Trennanlage ist insbesondere zur Durchführung eines Verfahrens eingerichtet, wie es zuvor erläutert wurde.

Diese Trennanlage, ebenso wie eine erfindungsgemäß vorgesehene Anlage zur Herstellung von Dimethylether, profitiert von den zuvor erläuterten Vorteilen, auf die daher ausdrücklich verwiesen wird.

Die Erfindung wird unter Bezugnahme auf die Zeichnungen näher erläutert, die eine Ausführungsform der Erfindung gegenüber dem Stand der Technik zeigen.

Kurze Beschreibung der Zeichnungen
Figur 1 zeigt eine Anlage zur Erzeugung von Dimethylether gemäß dem Stand der Technik in schematischer Darstellung
Figur 2 zeigt eine Anlage zur Erzeugung von Dimethylether gemäß einer Ausführungsform der Erfindung in schematischer Darstellung.

In den Figuren sind einander entsprechende Elemente mit identischen Bezugszeichen angegeben und werden der Übersichtlichkeit halber nicht wiederholt erläutert.

### Ausführliche Beschreibung der Zeichnungen

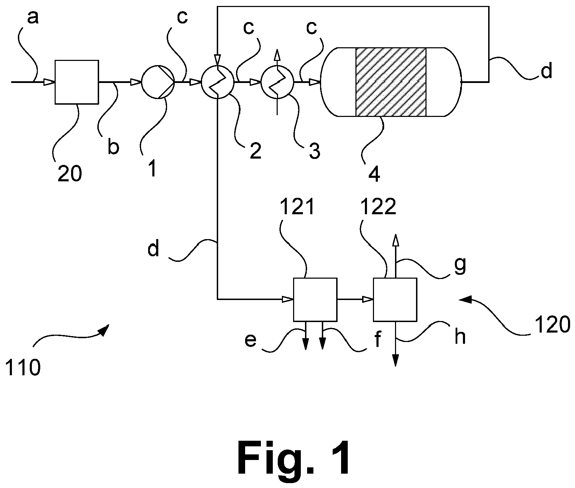

In Figur 1 ist eine Anlage zur Erzeugung von Dimethylether gemäß dem Stand der Technik schematisch dargestellt und insgesamt mit 110 bezeichnet.

Die Anlage 110 umfasst einen hier stark schematisiert dargestellten Synthesegasreaktor 20, der mit einem geeigneten Einsatz a, beispielsweise Erd- oder Biogas, beschickt werden kann. Dem Synthesegasreaktor 20 kann ein Synthesegasstrom b entnommen werden.

Der Synthesegasstrom b kann, gegebenenfalls nach einer Beimischung weiterer Ströme, mittels eines Verdichters 1 druckerhöht werden. Hierdurch kann ein für eine nachfolgende einstufige Dimethylethersynthese erforderlicher Druck, beispielsweise ein Druck von 20 bis 80 bar, eingestellt werden.

Ein entsprechend verdichteter Strom, nun mit c bezeichnet, wird durch einen ersten Wärmetauscher 2 geführt, der mit einem Produktstrom d eines Reaktors 4 zur Synthese von Dimethylether (siehe unten) erwärmt werden kann. Der entsprechend erwärmte Strom, weiter mit c bezeichnet, weist stromab des ersten Wärmetauschers 2 beispielsweise eine Temperatur von 200 bis 300 °C auf. Der Strom c wird gegebenenfalls durch einen zweiten Wärmetauscher 3 geführt, welcher auch als Spitzenerhitzer bezeichnet wird.

Der in dem zweiten Wärmetauscher 3 weiter erwärmte Strom, auch hier weiter mit c bezeichnet, wird in den Reaktor 4 eingespeist, der als Rohrreaktor ausgebildet ist und dessen Reaktionsrohre mit einem geeigneten Katalysator zur einstufigen Synthese von Dimethylether befüllt sind. Die Darstellung in der Figur 1 ist stark vereinfacht. Typischerweise sind Reaktoren 4 zur Synthese von Dimethylether stehend angeordnet, wobei ein Strom c bodenseitig in den Rohrreaktor 4 eingespeist wird. Kopfseitig wird dem Reaktor 4 ein Strom d entnommen.

Aufgrund der exothermen Reaktion in dem Rohrreaktor 4 liegt der Strom d bei nochmals höherer Temperatur vor. Der Strom d wird als Heizmedium durch den Wärmetauscher 2 geführt. Er kühlt sich hierdurch auf eine Temperatur ab, die beispielsweise ca. 30 °C oberhalb der Temperatur des Stroms c stromab des Verdichters 1 liegt. Der entsprechend abgekühlte Strom, weiter mit d bezeichnet, wird einer herkömmlichen Trennanlage 120 zugeführt. In der Trennanlage 120 werden aus dem Strom d beispielsweise unter zwischenzeitlicher Entspannung, Abkühlung, Rückverdichtung usw. (nicht dargestellt) in einem Schritt 121 ein Methanolstrom e und ein Wasserstrom f abgetrennt. Aus dem verbleibenden Rest werden die Ströme g und h gebildet, bei denen es sich beispielsweise um einen an Kohlenstoffdioxid angereicherten Strom g und einen an Dimethylether angereicherten Strom h handeln kann.

Die Zusammensetzung der Ströme g und h richtet sich unter anderem nach der Zusammensetzung des Stroms d und der spezifischen Ausgestaltung und den Betriebsparametern der Trennanlage 120.

Figur 2 zeigt eine Anlage zur Erzeugung von Dimethylether gemäß einer Ausführungsform der Erfindung. Diese ist insgesamt mit 100 bezeichnet.

Eine erste Absorptionskolonne, die zur Abtrennung von Methanol und/oder Wasser eingesetzt wird, ist in der Figur 2 mit 6 bezeichnet. Wie bereits erläutert unterscheidet sich eine Absorptionskolonne 6 von einer Destillationssäule wie der Destillationssäule 5 unter anderem dadurch, dass sie keinen Sumpfverdampfer aufweist. In der Absorptionskolonne 6 aufsteigende Dämpfe werden durch einen am Kopf der Absorptionskolonne aufgegebenen Rücklauf gewaschen, so dass sich am Kopf der Absorptionskolonne die leichter flüchtigen und im Sumpf der Absorptionskolonne die schwerer flüchtigen Komponenten anreichern.

In der Anlage 100, die in Figur 2 dargestellt ist, wird der Strom d in die Absorptionskolonne 6 eingeleitet. Vom Kopf der Absorptionskolonne 6 wird ein Kopfstrom k entnommen und in einem Wärmetauscher 7 gegen ein geeignetes Kältemittel, beispielsweise Kühlwasser, gekühlt. Der entsprechend abgekühlte Strom k wird in einen Abscheiderbehälter 8 überführt, aus dessen Sumpf ein flüssiger Strom I entnommen und mittels einer Pumpe (ohne Bezeichnung) zumindest zum Teil als Rücklauf auf die Absorptionskolonne 6 aufgegeben wird.

Enthält der Strom d im dargestellten Beispiel neben Dimethylether auch Methanol, Wasser, Kohlenstoffdioxid, Kohlenstoffmonoxid und Wasserstoff (neben Spuren anderer Verbindungen wie oben erläutert), gehen hiervon aufgrund der erläuterten Rückwaschung Dimethylether, Kohlenstoffdioxid, Kohlenstoffmonoxid und Wasserstoff in den Kopfstrom k über. Durch eine geeignete Abkühlung in dem Wärmetauscher 7 und entsprechende Abscheidebedingungen in dem Abscheiderbehälter 8 scheidet sich in dem Abscheiderbehälter 8 ein Sumpfprodukt ab, das im Wesentlichen aus Dimethylether und Kohlenstoffdioxid (gegebenenfalls mit Spuren von Methanol) besteht.

Vom Kopf des Abscheiderbehälters 8 kann ein Strom m gasförmig abgezogen werden, der zusätzlich zu Kohlenstoffdioxid, Kohlenstoffmonoxid und Wasserstoff ebenfalls noch Dimethylether enthält. Der Strom m wird anschließend einer sequenziellen Abkühlung und Kondensation unterworfen, wie unten erläutert. Der nicht als flüssiger Rücklauf auf die Absorptionskolonne 6 aufgegebene Anteil des Stroms I wird, wie die bei der sequenziellen Abkühlung und Kondensation des Strom m anfallenden Kondensate, in eine Destillationssäule 9, die hier als Dimethylether-Kohlenstoffdioxid-Destillationssäule 9 bezeichnet wird, eingespeist.

Es sei ausdrücklich betont, dass die spezifische Bereitstellung des Stroms k, der aus dem Produktstrom d gewonnen wird, nicht in der dargestellten Weise erfolgen muss. Auch andere Möglichkeiten zur Abtrennung von Wasser und/oder Methanol sind einsetzbar, sofern sie zur Erzeugung eines Gasgemischs auf dem zuvor erwähnten ersten Druckniveau und dem ersten Temperaturniveau und mit den angegebenen Gehalten an den einzelnen Komponenten führen.

Es ist beispielsweise auch möglich, auf die Destillationssäule 5 zu verzichten und Wasser auf andere Art und Weise aus dem Strom n abzutrennen. Insbesondere wird das Wasser also mit in die Dimethylether-Kohlenstoffdioxid-Destillationssäule 9 eingespeist. In solchen Fällen wird das Wasser ggf. in einer der Dimethylether-Kohlenstoffdioxid-Destillationssäule 9 nachgelagerten Anlagenkomponente aus dem Dimethylether-Produkt z abgetrennt.

Aus dem Sumpf der Absorptionskolonne 6 wird ein flüssiger Strom n entnommen und in geeigneter Höhe in die Destillationssäule 5 eingespeist, welche mit einem Sumpfverdampfer 51 und einem Kopfkondensator 52 betrieben wird. Der Strom n enthält im dargestellten Beispiel den überwiegenden Anteil des in dem Strom d enthaltenen Wassers und Methanols.

Der Sumpfverdampfer 51 und der Kopfkondensator 52 werden mit geeigneten, vorzugsweise in einer entsprechenden Anlage vorhandenen, Heiz- bzw. Kühlmedien betrieben. In dem Sumpfverdampfer 51 wird ein flüssiger, aus dem Sumpf der Destillationssäule 5 abgezogener Strom zum Teil verdampft und in einen unteren Bereich der Destillationssäule 5 eingespeist. Ein nicht verdampfter Anteil kann als Strom p abgezogen werden.

Vom Kopf der Destillationssäule 5 wird ein gasförmiger Strom abgezogen, zum Teil in dem Kopfkondensator 52 der Destillationssäule 5 verflüssigt und als flüssiger Rücklauf in einem oberen Bereich erneut in die Destillationssäule 5 eingespeist. Ein gasförmig verbleibender Anteil o wird abgezogen.

Aus dem Strom n, der im Wesentlichen noch Wasser, Methanol, Wasserstoff, Dimethylether und Kohlenstoffdioxid enthält, wird also in der Destillationssäule 5 ein im Wesentlichen Dimethylether und Kohlenstoffdioxid enthaltender Strom (Strom o) und ein im Wesentlichen Methanol und Wasser enthaltender Strom (Strom p) gebildet. Der Strom o kann an geeigneter Stelle in den Trennprozess zurückgeführt werden. Der Strom p kann anderweitig verwendet werden. Abgeschiedenes Wasser kann auch zu einer Abwasserbehandlung oder einer Entgasung geführt werden.

Die Destillationssäule 5 kann auch so betrieben werden, dass im Wesentlichen kein Wasser, jedoch Methanol in nicht zu vernachlässigender Menge in das Kopfprodukt o übergeht. Dies ist beispielsweise vorteilhaft, wenn der Dimethylether für Einsatzzwecke verwendet werden soll, bei denen Methanol bei der Verwendung nicht stört. So kann eine höhere Ausbeute bezogen auf das eingesetzte Synthesegas erzielt werden.

Rücklaufmenge und Bodenzahl der Absorptionskolonne 6 können so optimiert werden, dass ein entsprechendes Sumpfprodukt n in möglichst geringer Menge anfällt. Vorteilhafterweise wird der Rücklauf, der als Anteil des Stroms I auf die Absorptionskolonne 6 aufgebracht wird, derart eingestellt, dass der Methanol- und/oder Wassergehalt in dem Strom k minimiert wird. Die sich auf diese Weise ergebende Zusammensetzung des Stroms m ist derart, dass sich in der Abkühlungs- und Kondensationssequenz, der der Strom m unterworfen wird, die eingangs erläuterten Nachteile nicht mehr einstellen können.

Die bereits mehrfach erwähnten Schritte zur Weiterbehandlung des Stroms m sind hier insgesamt mit 10 angegeben. Der Strom m wird dabei zunächst einem Wärmetauscher 11 zugeführt und anschließend in einen Abscheiderbehälter 12 eingespeist. Die Abkühlung in dem Wärmetauscher 11 wird dabei derart vorgenommen, dass sich in dem Abscheiderbehälter 12 ein erstes Kondensat q abscheidet. Ein in dem Abscheiderbehälter 12 gasförmig verbleibender Anteil wird einem Wärmetauscher 13 zugeführt und anschließend in einen weiteren Abscheiderbehälter 14 eingespeist. Auch dort wird ein Kondensat, hier mit r bezeichnet, erhalten.

Die Kondensate q und r werden, zusammen mit dem nicht auf die Absorptionskolonne 6 rückgeführten Anteil des Stroms I, in die bereits erwähnte Dimethylether-Kohlenstoffdioxid-Destillationssäule 9 eingespeist, die wie unten erläutert betrieben wird. Ein auch am Kopf des Abscheiderbehälters 14 gasförmig verbleibender Anteil wird in einem weiteren Wärmetauscher 15 abgekühlt. Dieser Strom, hier mit s bezeichnet, liegt stromab des Wärmetauschers 15 auf dem mehrfach erläuterten "zweiten" Temperaturniveau knapp oberhalb des Schmelzpunkts von Kohlenstoffdioxid (bei dem vorherrschenden Druck) vor. Die Temperatur des Stroms m stromauf des Wärmetauschers 11 (also das "erste" Temperaturniveau) beträgt demgegenüber beispielsweise +35 °C. Der entsprechend abgekühlte Strom s wird in eine Absorptionskolonne 16 überführt, die erfindungsgemäß betrieben werden kann.

Die Erfindung kann auch in einer stark vereinfachten Anordnung zum Einsatz kommen, beispielsweise bei einer einstufigen Abkühlung, die der Abtrennung von Methanol und Wasser nachgeschaltet ist. Immer wird jedoch ein auf dem zweiten Temperaturniveau gasförmig verbleibender Anteil des Gasgemischs in einer Absorptionskolonne 16 mit einem überwiegend Dimethylether enthaltenden Rücklauf gewaschen, wie nachfolgend erläutert. Der überwiegend Dimethylether enthaltende Rücklauf wird dabei aus einem bei der Abkühlung abgeschiedenen flüssigen Anteil des Gasgemischs gebildet.

Der Strom s enthält im dargestellten Beispiel noch Dimethylether, Kohlenstoffdioxid, Kohlenstoffmonoxid und Wasserstoff, also neben Dimethylether und Kohlenstoffdioxid hier zwei leichter als Kohlenstoffdioxid siedende Komponenten. Unter Verwendung eines flüssigen Rücklaufs v, der reich an Dimethylether ist und aus einem Teil eines Kondensats z gebildet wird, das aus einem Sumpfstrom aus einer Sumpfflüssigkeit der Dimethylether-Kohlenstoffdioxid-Destillationssäule 9 erhalten wird, wird im Sumpf der Absorptionskolonne 16 ein Gemisch aus Dimethylether und Kohlenstoffdioxid abgeschieden und in Form des Sumpfprodukts w abgezogen. Das Sumpfprodukt w kann ebenfalls in die Dimethylether-Kohlenstoffdioxid-Destillationssäule 9 eingespeist werden. Am Kopf der Absorptionskolonne 16 wird hingegen ein Kopfprodukt x abgezogen, das im Wesentlichen aus Kohlenstoffmonoxid und Wasserstoff besteht und arm oder vorzugsweise frei an Kohlenstoffdioxid ist. Dieses wird, gegebenenfalls nach entsprechender Verdichtung in einem Verdichter 17, zumindest teilweise als Recyclingstrom j dem Strom b beigemischt.

Ein weiterer Teil des an Kohlenstoffdioxid abgereicherten Kopfprodukts x aus der Absorptionskolonne 16 kann als Purgestrom i aus dem Verfahren ausgeschleust werden.

Der Purgestrom i kann einer zusätzlichen Wäsche mit flüssigem Kohlenstoffdioxid unterzogen werden, um möglichst große Anteile des darin enthaltenen Dimethylethers in die Dimethylether-Kohlenstoffdioxid-Destillationssäule 9 zurückzuführen. Für diese Wäsche kann vorteilhafterweise ein Teil des kondensierten Kopfgases t aus der Dimethylether-Kohlenstoffdioxid-Destillationssäule 9 verwendet werden. Dies ermöglicht eine Verringerung der Verluste an Dimethylether, die andernfalls über die Ausschleusung des Purgestroms i entstünden. Da jedoch nur der Purgestrom i dieser zusätzlichen Wäsche unterzogen wird, wird hierdurch kein zusätzliches Kohlenstoffdioxid in den Recyclingstrom j eingetragen. In dem Recyclingstrom j enthaltener Dimethylether wird somit durch den Reaktor 4 in den Trennzyklus zurückgeführt und bleibt somit ebenfalls weitgehend erhalten.

In einigen Ausgestaltungen kann das Kopfgas x' der Absorptionskolonne 16 in einem optionalen Kopfkondensator 162 kondensiert und mit dem Strom v als Rücklauf zur Absorptionskolonne 16 verwendet werden. Dadurch wird der Anteil an Dimethylether im Kopfprodukt x reduziert, was zu einem verbesserten Reaktionsgleichgewicht und reduzierten Verlust über den Purgestrom i führt.

In die Dimethylether-Kohlenstoffdioxid-Destillationssäule 9 werden, wie erwähnt, der nicht in die Absorptionskolonne 6 rückgeführte Anteil des Stroms I sowie die Ströme q und r und das Sumpfprodukt w eingespeist. Da diese unterschiedliche Gehalte an Dimethylether und Kohlenstoffdioxid aufweisen (Spuren von Kohlenstoffmonoxid und Wasserstoff sind in gelöster Form ebenfalls enthalten), werden diese in unterschiedlicher Höhe in die Dimethylether-Kohlenstoffdioxid-Destillationssäule 9 eingespeist, wozu geeignete Ventile (ohne Bezeichnung) vorgesehen sind.

Auch die Dimethylether-Kohlenstoffdioxid-Destillationssäule 9 wird mit einem Sumpfverdampfer 91 und einem Kopfkondensator 92 betrieben. Ein aus einem Kopfgas der Dimethylether-Kohlenstoffdioxid-Destillationssäule 9 gebildeter Kopfstrom t wird in dem Kopfkondensator 92 unter Verwendung eines mit einem geeigneten Kältemittel betriebenen Wärmetauschers zumindest teilweise verflüssigt und als flüssiger Rücklauf auf die Dimethylether-Kohlenstoffdioxid-Destillationssäule 9 aufgegeben. Ein weiterer Anteil wird zur Bildung eines weiteren Stroms y verwendet, der anderweitig verwendet werden kann.

Aus dem Sumpf der Dimethylether-Kohlenstoffdioxid-Destillationssäule 9 wird ein flüssiger Strom z entnommen, der hier im Wesentlichen aus Dimethylether besteht, jedoch insbesondere frei oder arm an Kohlenstoffdioxid ist. Ein Teil dieses Sumpfstroms z wird zur Bildung des Rücklaufs v für die Absorptionskolonne 16 verwendet. Außerdem wird aus dem Sumpfstrom z das Dimethyletherprodukt gebildet.

Als der Rücklauf v kommt auch der kondensierte Kopfstrom o der Destillationssäule 5 in Frage, da er im Wesentlichen frei von Kohlenstoffdioxid und reich an Dimethylether und ggf. Methanol ist.

Wird Wasser, wie oben in Bezug auf eine Ausführungsform beschrieben, erst stromab der Dimethylether-Kohlenstoffdioxid-Destillationssäule 9 abgetrennt, ist es zweckmäßig, einen im Wesentlichen wasserfreien Seitenstrom der Dimethylether-Kohlenstoffdioxid-Destillationssäule 9 anstelle deren Sumpfprodukts z als den Rücklauf v für die Absorptionskolonne 16 zu verwenden. Dabei wird der Seitenstrom so gewählt, dass er weniger als 10 Massenprozent, insbesondere weniger als 5 Massenprozent Kohlenstoffdioxid enthält.

Der Rücklauf v wird vor der Aufgabe auf die Absorptionskolonne 16 gekühlt, typischerweise auf ein Temperaturniveau, das bei den gewählten Bedingungen etwas über dem Gefrierpunkt von Kohlenstoffdioxid liegt, beispielsweise auf ein Temperaturniveau im Bereich von -30 °C bis -49 °C. Zu diesem Zweck kann beispielsweise ein Wärmetauscher 164 vorgesehen sein.

## Patentansprüche

1. Verfahren zur Herstellung von Dimethylether (Abkürzung: DME) aus Synthesegas, wobei ein Einsatz (c), der unter Verwendung von geshiftetem und/oder ungeshiftetem Synthesegas (b) gebildet wird, unter Bildung eines Produktstroms (d) einer katalytischen Umsetzung unterworfen wird, wobei der Produktstrom (d) einer ersten Auftrennung unterworfen wird, wobei ein Gasgemisch (k) durch zumindest teilweise Abtrennung von Methanol und/oder Wasser aus dem Produktstrom (d) gebildet wird, und wobei das Gasgemisch (k) auf einem ersten Druckniveau durch Abkühlung von einem ersten auf ein zweites Temperaturniveau teilweise kondensiert wird, **dadurch gekennzeichnet, dass** ein auf dem zweiten Temperaturniveau gasförmig bleibender Anteil (s) des Gasgemischs (k) in einer Absorptionskolonne (16) mit einem überwiegend Dimethylether enthaltenden Rücklauf (v) gewaschen wird, wobei der überwiegend Dimethylether enthaltende Rücklauf (v) zumindest teilweise aus einem bei der Abkühlung kondensierten Anteil des Gasgemischs (k) gebildet wird, und wobei der in der Absorptionskolonne (16) nicht ausgewaschene gasförmige Anteil (x) des Gasgemischs (k) zumindest teilweise in den Einsatz (c) überführt wird und ein Dimethyletherprodukt (z) unter Verwendung des bei der Abkühlung kondensierten Anteils (l, q, r, w) des Gasgemischs (k) gebildet wird.

2. Verfahren nach Anspruch 1, bei dem das Gasgemisch (k) über mehrere Zwischentemperaturniveaus auf das zweite Temperaturniveau abgekühlt wird, wobei mehrere Kondensate (l, q, r, w) abgeschieden werden.

3. Verfahren nach einem der vorstehenden Ansprüche, bei dem der auf dem zweiten Temperaturniveau gasförmig bleibende Anteil (s) des Gasgemischs (k) in der Absorptionskolonne (16) unter Gewinnung eines Kopfprodukts (x) und eines Sumpfprodukts (w) mit dem überwiegend Dimethylether enthaltenden Rücklauf (v) gewaschen wird, wobei der überwiegend Dimethylether enthaltende Rücklauf (v) zumindest teilweise aus dem Sumpfprodukt (w) und/oder teilweise aus dem Kopfprodukt (x) gebildet wird.

4. Verfahren nach einem der vorstehenden Ansprüche, bei dem der überwiegend Dimethylether enthaltende Rücklauf (v) unter Verwendung einer Dimethylether-Kohlenstoffdioxid-Destillationssäule (9) gebildet wird.

5. Verfahren nach Anspruch 4, bei dem die Dimethylether-Kohlenstoffdioxid-Destillationssäule (9) derart betrieben wird, dass sich an ihrem Kopf ein überwiegend Kohlenstoffdioxid enthaltendes Kopfgas und in ihrem Sumpf eine an Dimethylether angereicherte Sumpfflüssigkeit bilden.

6. Verfahren nach Anspruch 5, bei dem als der überwiegend Dimethylether enthaltende Rücklauf (v) ein Teil des Dimethyletherprodukts (z) verwendet wird, das aus zumindest einem Teil der Sumpfflüssigkeit der Dimethylether-Kohlenstoffdioxid-Destillationssäule (9) gebildet wird.

7. Verfahren nach Anspruch 5 oder 6, bei dem zumindest ein Teil der Sumpfflüssigkeit der Dimethylether-Kohlenstoffdioxid-Destillationssäule (9) als Dimethyletherprodukt (z) abgezogen wird, der einen Dimethylethergehalt von mehr als 90 Molprozent, insbesondere mehr als 95 Molprozent, insbesondere mehr als 98,5 Molprozent aufweist.

8. Verfahren nach einem der Ansprüche 4 bis 7, bei dem die Dimethylether-Kohlenstoffdioxid-Destillationssäule (9) auf einem zweiten Druckniveau betrieben wird, das unterhalb des ersten Druckniveaus liegt.

9. Verfahren nach einem der vorstehenden Ansprüche, bei dem der überwiegend Dimethylether enthaltende Rücklauf (v) einen Kohlenstoffdioxidgehalt von höchstens 5 Gewichtsprozent, insbesondere höchstens 2 Gewichtsprozent, höchstens 1 Gewichtsprozent, höchstens 0,5 Gewichtsprozent oder höchstens 0,1 Gewichtsprozent aufweist.

10. Verfahren nach einem der vorstehenden Ansprüche, wobei ein nicht in den Einsatz (c) überführter Anteil des nicht ausgewaschenen gasförmigen Anteils (x) des Gasgemischs (k) als ein Purgestrom (i) teilweise aus dem Verfahren ausgeschleust wird, wobei der Purgestrom (i) unter Erhalt eines Waschstroms einer Wäsche mit zumindest teilweise flüssigem Kohlenstoffdioxid, das insbesondere unter Verwendung der Dimethylether-Kohlenstoffdioxid-Destillationssäule (9) bereitgestellt wird, unterzogen wird und der Waschstrom zumindest teilweise in die Dimethylether-Kohlenstoffdioxid-Destillationssäule (9) zurückgeführt wird.

11. Verfahren nach einem der vorstehenden Ansprüche, bei dem das erste Temperaturniveau 10 bis 50 °C, insbesondere 20 bis 40 °C beträgt und/oder bei dem das zweite Temperaturniveau 0,5 bis 20 °C, insbesondere 1 bis 10 °C oberhalb der Schmelztemperatur von Kohlenstoffdioxid bei dem ersten Druckniveau liegt, und/oder bei dem das erste Druckniveau 20 bis 100 bar, insbesondere 30 bis 80 bar beträgt.

12. Verfahren nach einem der vorstehenden Ansprüche, wobei das Synthesegas (b) über die Absorptionskolonne (16) zusammen mit dem nicht ausgewaschenen gasförmigen Anteil (x) des Gasgemischs (k) in den Einsatz (c) überführt wird.

13. Verfahren nach einem der vorstehenden Ansprüche, wobei ein Kopfgas (x') der Absorptionskolonne (16) zumindest teilweise kondensiert wird und der kondensierte Anteil des Kopfgases x' zumindest teilweise in den Kopfbereich der Absorptionskolonne (16) zurückgeführt wird.

14. Trennanlage, die zur trenntechnischen Bearbeitung eines Gasgemischs (k) eingerichtet ist, das aus einem Produktstrom (d) eines Reaktors (4) zur Synthese von Dimethylether aus Synthesegas (b) gebildet werden kann, und das zumindest Dimethylether, Kohlenstoffdioxid und zumindest eine weitere, leichter als Kohlenstoffdioxid siedende Komponente enthält, mit Mitteln, die dafür eingerichtet sind, das Gasgemisch (k) unter zumindest teilweiser Abtrennung von Methanol und/oder Wasser aus dem Produktstrom (d) bereitzustellen und auf einem ersten Druckniveau von einem ersten auf ein zweites Temperaturniveau abzukühlen und einen auf dem zweiten Temperaturniveau gasförmig bleibenden Anteil (s) des Gasgemischs (k) in einer Absorptionskolonne (16) mit einem überwiegend Dimethylether enthaltenden Rücklauf (v) zu waschen, wobei ferner Mittel vorgesehen sind, die dafür eingerichtet sind, den überwiegend Dimethylether enthaltenden Rücklauf (v) zumindest teilweise aus einem bei der Abkühlung kondensierten Anteil (l, q, r, w) des Gasgemischs (k) zu bilden.

15. Anlage (100) zur Herstellung von Dimethylether, mit zumindest einem zur Synthese von Dimethylether aus Synthesegas (b) eingerichteten Reaktor (4) und einer Trennanlage nach Anspruch 14.
